# EUROPEAN PATENT SPECIFICATION

(11) **EP 1 399 082 B1**
(45) Date of publication and mention of the grant of the patent: **01.10.2008**
(21) Application number: 01963328.8
(22) Date of filing: 27.06.2001
(51) Int. Cl.: A61F 2/00, A61B 17/064

(54) **ADJUSTABLE AUTOFIXING SLING FOR TREATMENT OF URINARY INCONTINENCE**
VERSTELLBARE SELBSTFIXIERENDE SCHLINGE ZUR BEHANDLUNG VON HARNINKONTINENZ
AGRAFE AUTOFIXANTE REGLABLE UTILE DANS LE TRAITEMENT DE L'INCONTINENCE URINAIRE

(43) Date of publication of application: 24.03.2004
(73) Proprietor: Promedon Do Brasil Produtos Medico-Hospitalares Ltda, 01333-010 Bela Vista - SP (BR)
(72) Inventor: FIERRO, Eduardo, 5000 Cordoba (AR)
(74) Representative: Casalonga, Axel
(86) International application number: PCT/IB2001/001642
(87) International publication number: WO 2003/002027

(56) References cited:
- EP-A- 0 632 999
- WO-A-00/74633
- WO-A-98/35632
- FR-A- 2 787 990
- US-A- 5 647 836
- US-A1- 2001 000 533
- US-B1- 6 221 005

## Description

The present invention relates to a sling for treating urinary incontinence in women and men. It concerns more particularly a sling consisting of a band which comprises a middle part and two end parts, the middle part being perforated and the two end parts constituing an autofixing system, and a kit which contains such a sling in a sterile manner.

Urinary incontinence affects many people, men after prostatectomy, and mainly women. Four types of urinary incontinence have been defined by the International Continence Society : stress, urge, overflow and reflex incontinence.

The first type and the more frequent, called stress incontinence, takes place during straining, following laughing or coughing, or during physical exercise. It results from weakness of the urethral sphincter which is no longer able to seal off the bladder, due to a loosening of the muscles of the perineum and/or Intrinsic Sphincter Deficiency (ISD). This form of incontinence can occur after childbirth or at the menopause, but it can also affect young sportswomen overdeveloping their abdominal muscles to the detriment of the perineum or in neurogenyc bladder such as myelomeningocele.

The second more frequent form of incontinence, referred to as overactive bladder, results from involuntary contractions of the (hyperactive) bladder and is manifested in an excessively frequent and irrepressible urge to urinate.

Some women suffer from mixed incontinence, which is a combination of the forms mentioned above.

Overactive bladder can be cured by taking medication aimed at relaxing the bladder.

For treating stress incontinence or preventing this incontinence, it is often necessary to resort to surgery.

The techniques known from the prior art consist in restoring the natural mechanisms of continence: in maintaining the urethra in the abdominal cavity and/or in increasing urethral resistency. To do this, a sling has already been used which is placed under the bladder neck or under the urethra, thereby making it possible to improve the suspension and some compression of the bladder neck and/or of the urethra.

Thus, application WO 98/35632 describes a stabilization sling for use in minimally invasive pelvic surgery and designed for urethral suspension, and US patent 5 934 283 concerns a pubovaginal sling device.

The patent application WO 00/74633 describes a method and a device for the implantation of a tape mesh for urethral suspension using a minimally invasive approach.

However, the slings used hitherto may, in some cases, cause friction in the area of the vagina, urethra or bladder. The reason for this is that during movements, the said slings may injure the different organs with which they are in contact. This friction in the area of the vagina, urethra or bladder may then cause erotions, inflammations or infections, or even cause rejection of the sling and make it necessary to remove the said sling, and, consequently, to perform a new operation.

It has thus been found that it is necessary, in one woman in every seven in the highest series of complications, to proceed with a new operation for removing the said sling. The percentage of sling extraction due to rejection and erosion ranks from 3 to 22%. This is sustained by the following references:
- D.Myers and C. La Sala, "Conservative surgical management of mersilen mesh suburethral sling erosion", AM.J.OBSTET.GYNEC., December 1998-Vol 179, n°6, part 1.
   - - Summit, "Suburethral sling procedure for genuine stress incontinence and low urethral clossure pressure: a continued experience", UROGYNECOL.J-1992, 3, 18-21.
   - - Weinberger, Mostergard D., "Long term clinic and urodynamic outcomer of PTFE suburethral sling for treatment of genuine stress incontinence", OBSTET.GYNECOL-1995, 86, 92-6.
- Bent, Ostergard, Zwick, Zafutto," Tissue reaction to expanded PTFE suburethral sling for urinary incontinence: clinical and histologic study", AM.J.OBSTET.GYNEC., 1993-Vol 169,1198-2004.
   - - Young et al, "The mersilene mesh suburethral sling: a clinical and urodynamic evaluation", AM.J.OBSTET.GYNEC., December 1995-Vol 173, n°6.
   - - M.Corujo, G.Badlani, "The use of synthetic material in the treatment of women with SUI lends strength and durability", CONTEMPORARY UROLOGY, March 1999, vol.11, n°3-PP, 76-81
   - - Yue Kim Chin, Stuart Stanton, "A follow up of silastic sling for genuine stress incontinence", BRITISH JOURNAL OF OBSTETRICS AND GYNAECOLOGY, February 1995, vol 102, 143-147.
   - - J. Kersey, "The gauze hammock sling operation in the treatment of stress incontinence" , British Journal of Obstetrics & Gynaecology, October 1983. Vol.90 pp: 945-949.-
   - - A. Korda, B. Peat and P. Hunter, "Silastic Sling for Female Incontinence", International Urogynecol Journal (1990) 1: 66-69.-

Other postoperative problems mentioned in the above articles and many other publications are:
a) Voiding difficulties: They normally occur when a sling is implanted with tension; that is to say, when there is an excess of urethral compression caused by the sling. The regulation of tension during surgery is a task to be performed in a skillfully way. The same happens when placing a sling which simply lies on the urethra (tension free). When the urethral or vesical neck compression is excessive, the patient has to do a huge effort to urinate and even when achieving it, there is a large amount of residual urine. Because of this, a new operation is needed and in the case of synthetic meshes, the sling has to be withdrawn or else the part of it that lies on the urethra has to be cut. If so, the patient becomes incontinent again. In the case of synthetic slings made of microporous meshes or blind straps, a suprapubic surgery is realized to correct the tension; releasing the sutures and suturing again with less tension. This is not always easy, and long incissions are performed until the sutures are eventually found.
b) Urinary incontinence: When the sling is placed loosely the patient is not cured (dry) but "improved", that is to say that there is fewer urine leakage than before the operation. To improve the continence a new surgery is required, in which the sling has to be tightened. In the case of a blind-strap sling, the surgery is suprapubic, similar to what it is explained in the above item a). In the case of a mesh-sling, the problem is more serious since fibrous tissue grows amongst the holes of the mesh, immobilizing it. The solution then is a major surgery to remove the mesh and replace it for another one, or to place a new sling on the former one.

The aim of the present invention is to propose novel slings which avoid these disadvantages, that is to say slings which are better tolerated by the body and thus have less risk of erosion than those in the prior art, and that allow with a minimally invasive approach the correction of the post-operative problems of voiding difficulties and urinary incontinence. This can be translated as obvious benefits for patients, since the post-operative problems can be corrected without removing the slings.

The subject of the present invention is therefore a sling for treatment of urinary incontinence, consisting of a band which comprises a middle part and two end parts, the middle part being perforated and the two end parts constituing an autofixing system.

The middle perforated part extends longitudinally between the two end part, when placed in the body, this middle part lies on the bladder neck or on the urethra ; amongst its holes or perforations grows the interconnective tissue between the vaginal flap and the urethra, which leads to a great integration of the implant without a loss of vascularization between the bladder and the vagina. As a matter of fact, since there is a normal vascularization in the area of the implant integration, the risk of necrosis and post-operative erosion/infection diminishes.

The two end parts of the sling constitute a system, which can be autofixed to the abdominal fascia, therefore the sling is fixed without sutures, the two end parts of the autofixing system cooperate by means of two complementary elements. In an advantageous manner this autofixing is enough to keep the sling in its place when there is an important muscular activity, such as coughing or other strains. When installed in the body, the two end parts are joined, nevertheless they can be displaced (adjusted) after surgery to correct the possible post-operative problems of urinary incontinence and voiding difficulties.

Urinary incontinence can be corrected by performing just a suprapubic punction (in only one of the sides) until the end of the said joined parts is found. It is then catched with a pair of pliers and the joined end parts are displaced by pulling it up softly, so the sling is fitted upon the surgeon's wish. This can be carried out while the bladder is full and making the patient cough in order to achieve the desired grade of continence.

Voiding difficulties can be solved in the same way that urinary incontinence, but pushing the joined end parts in. An alternative, is to perform a vaginal punction up to the joint between the perforated middle part and the joined end parts, to catch the joined end parts with a pair of pliers and pull it out until the urethra is no longer compressed.

According to the invention, the material from which the perforated middle part (2) is made can be any biocompatible biological or synthetic material. It can be filamentous or non-filamentous, elastic or non-elastic, porous or microporous. This material may be chosen depending on the properties sought, in particular to facilitate the surgical intervention and prevent any risk of rejection.

According to one embodiment of the invention, the perforated middle part is preferably made of silicone. In a preferred embodiment, this middle part contains a reinforcement which, according to a particular embodiment of the invention, consists of a polyester mesh.

The middle part can also be made of other synthetic materials such as polyesters, polypropylenes, polyurethanes, polyamides, nylons, silicones, polytetrafluoroethylenes such as Teflon, polyethylene terephthalates, latex or any other thermo-hardened or thermo-formed plastics or gums. In the case of fibrous materials, the latter may be woven or non-woven. Thus, it will be possible to use polyester meshes. It will also be possible to use a mixture of these different materials, for example a mixture of a silicone and Dacron, or a silicone reinforced by a polyester mesh.

In addition, the middle part can be made of biologic materials such as bovine pericardium, different types of collagen, or other animal or human derivatives such as processed fascia lata (Tutoplast®), porcine small intestinal submucosa (STRATASIS™), tissue regeneration matrix (REPLIFORM™), and suchlike.

The middle part (2) upon which the urethra lies, has a length of 20 - 80 mm, preferably 47 mm ; a width of 7 - 25 mm, preferably 15 mm and a thickness of 0.2 - 3.0 mm, preferably 0.5 mm.

The middle part of the sling has perforations throughout its surface, which are preferably circular but that can also be oval, rectangular, square, circularor any combinaison of these shapes, they also may be of varied rhombic shapes or of multiple geometric shapes (e.g. : star, etc). The said perforations in the middle part are preferably symmetrically distributed, following a predetermined pattern, but can also be randomly distributed. The diameter of the perforations is 4 times as great as the thickness of the middle part, but can be between 0,5 and 10 times greater. The variety of shapes in the perforations is aimed at obtaining different degrees of rigidity for the middle part.

Amongst the said holes or perforations grows the interconnective tissue between the vaginal flap and the urethra, which leads to a great integration of the implant without a loss of vascularization between the bladder and the vagina. As a matter of fact, since there is a normal vascularization in the area of the implant integration, the risk of necrosis and post-operative erosion/infection diminishes.

The perforated middle part has certain elasticity bestowed by the silicone. This property is enhanced because of the perforations (4) that, as a whole, offer a slightly elastic surface against which the urethra may lean, avoiding hence difficult micturitions and minimizing the possibility of urethral erosion.

The said middle part is preferably made of silicone and reinforced with a Dacron-mesh reinforcement, thus the middle part has the advantage of being a little elastic.

The reinforcement mesh may be or not necessary when the middle part is made of other syntethic materials (a polytetrafluoroethylene or a polypropylene) or of biologic materials (a collagen, bovine pericardium, processed fascia lata, etc).

The autofixing end parts are preferably made of silicone, or any other synthetic material such as a polypropylene, a polyurethane, a polytetrafluoroethylene such as Teflon, a polyamide or any other thermo-hardened or thermo-formed plastics or gums. Preferably these end parts are reinforced by means of Dacron

The end parts and the middle part of the sling are preferably radioopaque, but they can also be radiotranslucent.

The joining of each end part and the middle part is made using biocompatible adhesive, inserting the end of the middle part into the slot (8) that is in the proximal extremities of the end parts.

Synthetic biocompatible adhesives, preferably a silicone adhesive, may be used to join the end parts and the middle part, natural or biological adhesives may be used as well, such as a collagen-based adhesive

In the autofixing system, at least one end part is made of multiple subunits, the shape of the subunits may be conical, triangular, rectangular, square, trapezoidal, rhomboidal, oval, cylindrical. Preferably at least one end part is made of cone-shaped subunits. More preferably, the two end parts are made of cone-shaped or multispheric-shaped subunits.

The basis of the subunits may be straight or else fish hook-shaped. The end parts can also be multispheric, it can be spheres of the same or of different sizes, interchanging those sizes all along the end parts. These two end parts constitute an autofixing system which anchors at the abdominal wall, and therefore the sling is autofixed. There are infinite designs for the end parts, combining the different shapes and sizes the subunits may have. The variety of sizes and shapes in the subunits is aimed at obtaining different degrees of anchorage of the end parts to the abdominal wall and also different degrees of elasticity / elongation.

Each end part has a length of 100 - 300 mm, preferably about 175 mm.

Each subunit of the end parts has a minor diameter of 0.5 - 4.0 mm, preferably 3 mm ; a major diameter of 2 - 8 mm, preferably 5 mm and a height of 1 - 15 mm, preferably 4 mm. The said subunits are preferably of symmetrical shape that means their basis is circular but the basis can also be elliptical. In the latter case, the subunits that make up the end parts are elliptical cone-shaped. The subunits may also be trapezoidal, thus giving the end parts a flat aspect rather than cylindrical.

The end parts (3) are preferably opaque to X-Rays and they have certain elasticity which leads to normal micturitions and diminishes the risk of tissular erosion.

At the extremity of at least one of the end parts, preferably both, there is a perforation so that the end part can be threaded through a crochet-like needle, in order to pass the sling from the vagina to the suprapubic region. An alternative is to pass a thread through the perforation of the end part, thread a needle or catch it with a pair of pliers and transfer the sling up.

The shape particularly conical of the subunits that form each end part makes them fix by themselves to the muscles, thus immobilizing the sling. This autofixing of the sling is improved after the implantation surgery because of the resulting fibrosis that eventually wraps the whole sling.

The said sling can be entirely or partially impregnated with or covered by an antibiotic, antimicrobial agent or a combination of both that reduces the risk of intraoperative infection/contamination, such as a Silver coating, Chlorhexidine, Heparine or an Antibiotic Delivery System (MEDI-COAT™), and suchlike.

The implantation of the sling is performed by suprapubic approach, using a needle for suprapubic punction, which is guided by a finger into the retropubic space. The surgical technique is mentioned in many of the previously listed references. The steps of the surgical procedure for implanting the sling are the following :
1- Anterior colpotomy : Medium colpotomy or U colpotomy inverted at half distance between the meatus and the vesical neck, Raz-like, (Raz, S. : « Modified Bladder Neck Suspension for Female Stress Incontinence » - Urology, 17: 82, 1981.-) with development of vaginal flap.
2- Paraurethral dissection : Paraurethral passage to the retropubic space Raz-like (with scissors and finger), with acute and blunt dissection.
3- Sling transference to the suprapubic region : Scalpel puncture of skin over the pubis next to the superior edge, at 2 or 3 centimeters of each side of the medium line. Puncture separations : approximately 5-6 centimeters. Puncture passage, very close to the pubis, of pliers or an ad hoc needle (crochet-like) which will be received and guided by the surgeon's finger. Threading of an end part to the needle, passing the needle through the end part perforation. Transference of the end parts to the suprapubic region
4- Tension-free fixation : With a cytoscopic placed in the urethra, keeping a 30° angle regarding the horizontal, and once both end parts have been passed, they need to be pulled up until the middle part of the sling simply lies on the urethra without any tension. Sometimes, a pair of pliers is put between the middle part and the urethra to ensure the tension-free fitting.
5- Cutting the surplus of end parts : The fascia and abdominal muscles are slightly pressed and the surplus of both end parts is cut close to the tissue. Nevertheless a little surplus may be kept in order to adjust the two end parts after surgery. Punctions are then stitched and so is the colpotomy.

Figure 1 is a plan view of a sling according to the invention.
Figure 2 is a diagrammatic longitudinal section through a sling according to a preferred embodiment of the invention.
Figure 3 is a view of a sling section to show the autofixing to the abdominall wall.
Figures 4, 5 and 6 show different end parts made of multiple different-shaped autofixing subunits.
Figures 7 and 8 show the perforations of the middle part that lies on the urethra and their different shapes and patterns.
Figure 9 shows an ad-hoc needle, crochet-like, to transfer the ends of the end parts of the sling from the vaginal to the suprapubic area.

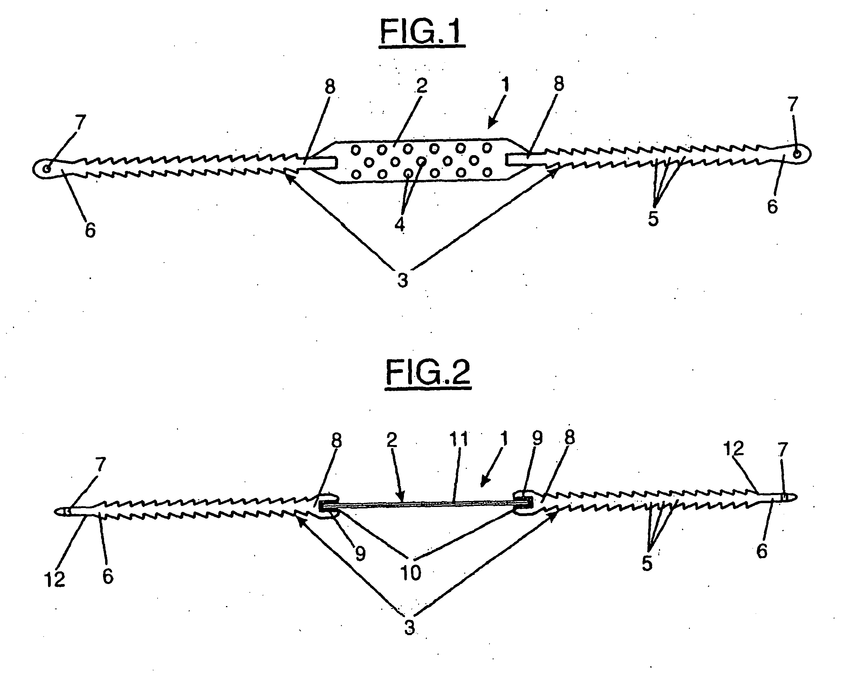

Figure 1 is a plan view of a sling (1) according to the invention. This sling consists of a perforated middle part (2) and two end parts (3) which are made of multiple cone-shaped subunits. The perforations are represented by the mark (4).

The autofixing end parts of the sling are made up of multiple cone-shaped subunits (5) which anchor at the abdominal wall, and therefore the sling is autofixed. At the extreme (6) of each end parts there is a perforation (7), through which the end of a crochet-like needle ad hoc can be passed. Such needle is used during surgery, when transfering the extremes of the end parts (6) from the vaginal to the suprapubic area.

The joining of each end part and the middle part is made using biocompatible adhesive, inserting the end of the middle part into the slot (8) that is in the proximal extreme of the end parts.

Figure 2 is a diagrammatic longitudinal section through a sling (1) according to a preferred embodiment of the invention. In this figure it is possible to see the joint between the middle part (2) and the proximal extremities (8) of both end parts (3) each constituted with cone-shaped subunits (5). Such extremes have a slot (9) into which the end of the middle part is inserted and fixed with an adhesive (10).

The perforated middle part (2) has a reinforcement (11) that increases its resistance.

The extremes of the autofixing system (6) are pointed (12), so that an ad hoc needle can pass through the perforation (7) in the said extremities.

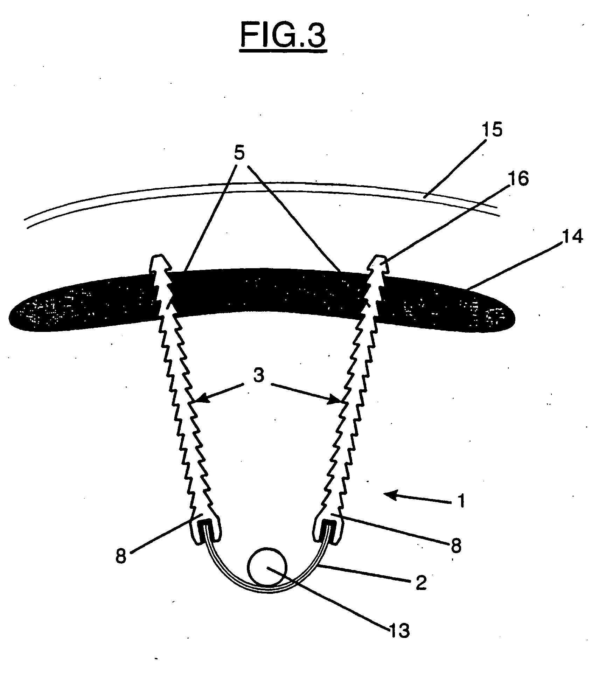

Figure 3 is a view of a sling section to show the autofixing to the abdominall wall. The figure shows the anatomic location of the sling (1). The perforated middle part (2) lies on the urethra free of tension (13), while the extremities of the autofixing end parts (3) are under the skin (15), once the surplus has been removed.

The cone-shaped subunits (5) that make up the end parts of the sling fix to the abdominal wall (14) by themselves. This autofixing is enough to keep the sling in its place when there is an important muscular activity, such as coughing or other strains.

The main advantage of the said autofixation system is to allow the surgeon to regulate the tension on the urethra, once the sling has been long implanted, and with a minimally invasive approach. To achieve this, the surgeon realizes a punction on the skin (15) with a scalpel, then takes the edge (16) of a end parts with a pair of pliers and moves the end parts upwards so as to tighten the sling around the urethra, or down to release it. Another option to lessen the tension on the urethra is to make an incission on the vaginal wall until finding the proximal extremity (8) of one of the end parts, and with a pair of pliers pull out such extremity.

The displacement of end parts when pulling them is of 4 mm at a time, because this is the height of a single cone-shaped subunit (5). This is the reason why the fitting of the autofixable sling is so precise.

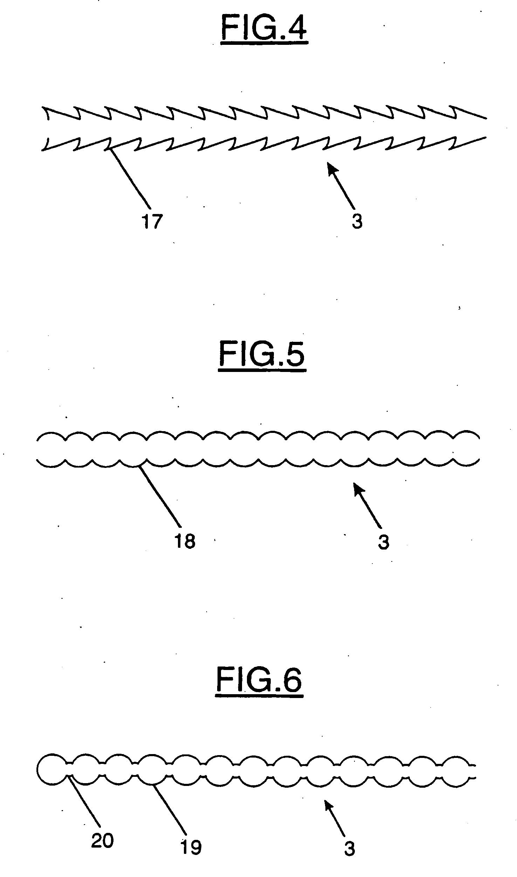

Figures 4, 5 and 6 show different end parts (3) made up of multiple different shaped autofixing subunits. In these figures, only a few alternatives of end parts made up of subunits of different sizes and shapes are shown.

Figure 4 shows an end part (3) made up of multiple fish-hook-shaped cones (17)

In Figure 5, the end part (3) consists in spherical subunits (18).

In figure 6, the end part (3) is made up of a combination of alternated little spheres (20) and larger spheres (19).

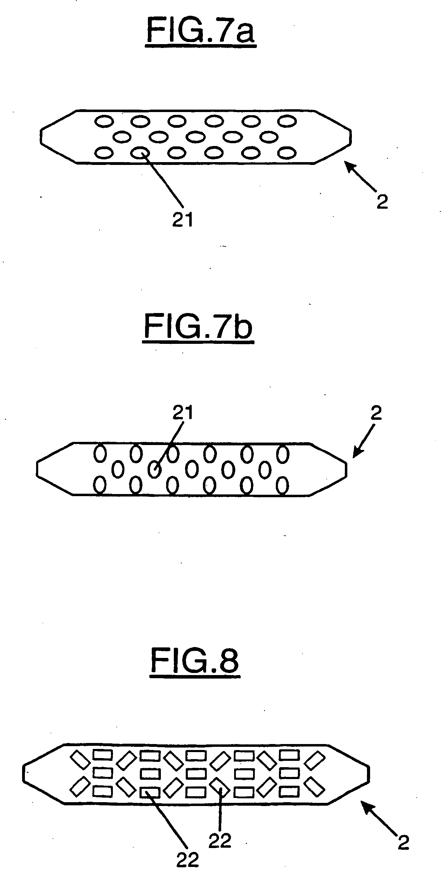

Figures 7a, 7b and 8 show some of the alternatives for the perforations of the middle part (2) and their different shapes and patterns. There are infinite designs for the perforated middle part, combining the different shapes and distributions (symmetrical or random) the perforations may have.

In figures 7a and 7b, the shown perforated middle parts (2) have both oval perforations (21), but they are not equally distributed. Because of this, both middle parts have different elasticity. The middle part in figure 7b is more flexible longitudinally than the middle part in figure 7a.

In figure 8, the perforations of the middle part (2) are rectangular (22). The particular distribution of these perforations increases the longitudinal flexibility / elongation of the middle part.

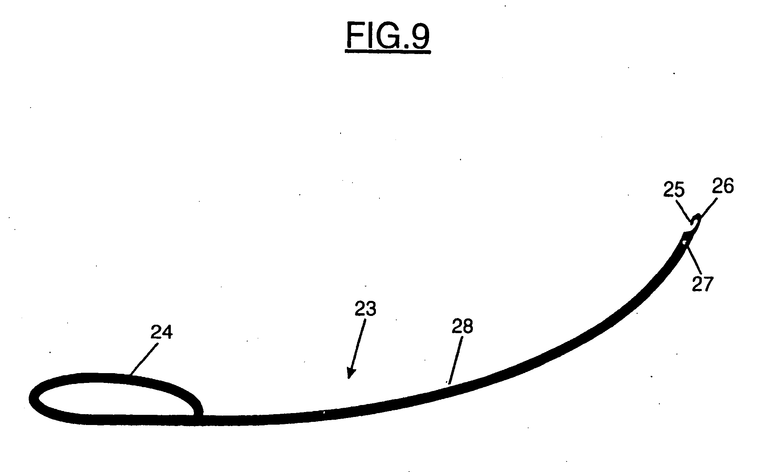

Figure 9 shows an ad-hoc needle that is used for implanting the sling. The said needle has two main uses : a) for suprapubic punction, in order to go through the abdominal wall from the suprapubic space up to the vagina ; and b) for transfering the extremes of the autofixing end parts of the sling to the suprapubic region.

The said needle (23) consists of a a cylindrical metallic rod, preferably a stainless steel rod, having a diameter of 2 - 6 mm, preferably 4 mm. In one of its extremities, it has a loop (24) for handling the needle and the other extremity is hooked, like a crochet needle (25), to hitch the extremity of the sling end part onto it, making the needle pass through the perforation (27) in the extremity of the end part.

The edge of the hooked (26) needle is not sharp or pointed. It does not even pierce the glove of the surgeon.

The diameter of the said crochet-like hook which is in one extreme of the needle is the same as the diameter of the metallic rod of the needle, or smaller.

The edges of the hook are rounded enough so that tissues are not grabbed when the needle passes through the abdominal fascia.

Close to the crochet-hook there is a hole (27) which diameter is between 0.7 and 1.5 mm. A thread can eventually be passed through it to help passing the end parts of the sling, previously tied to the thread. This is intended as a solution in case the perforation at the end of the end part breaks because of an excessive strain during surgery.

The curvature radius (28) of the needle is the one convenient for passing the said needle from the suprapubic to the vaginal region, through the retropubic space.

## Claims

1. Sling (1) for treatment of urinary incontinence, consisting of a band which comprises a middle part (2) and two end parts (3), the said middle part being perforated and **characterized in that** the two end parts constituting an autofixing system and **in that** the two end parts are made of mutiple subunits, the shape of these subunits being selected in the group consisting in conical, triangular, rectangular, square, trapezoïdal, rhomboidal, oval, cylindrical or multispheric shape.

2. Sling according to claim 1, **characterized in that** the basis of the subunits is straight or fish-hook-shaped.

3. sling according to any one of the preceding claims, **characterized in that** the middle part (2) is made up of a biocompaptible biological or a biocompaptible synthetic material.

4. Sling according to claim 3 **characterized in that** the middle part (2) is made of synthetic materials such as polyesters, polypropylenes, polyurethanes, polyamides, nylons, silicones, polytetrafluoroethylenes such as Teflon, polyethylene terephthalates, latex or any other thermo-hardened or thermo-formed plastics or gums; or biological materials such as bovine pericardium, different types of collagen, or other animal or human derivatives such as processed fascia lata, porcine small intestinal submucosa, tissue regeneration matrix.

5. Sling according to claim 4, **characterized in that** the middle part (2) is made of silicone.

6. Sling according to any one of the preceding claims, **characterized in that** the middle part contains a reinforcement preferably consisting of a polyester mesh.

7. Sling according to any one of the preceding claims, **characterized in that** the end parts are made of a material such as polyesters, polypropylenes, polyurethanes, polyamides, nylons, silicones, polytetrafluoroethylenes such as Teflon, polyethylene terephthalates, latex or any other thermo-hardened or thermo-formed plastics or gums,

8. Sling according to any one of the preceding claims, **characterized in that** at least one of the and parts contain a perforation.

9. Sling according to any one of the preceding claims, **characterized in that** it is entirely or partially impregnated with or covered by an antibiotic, an antimicrobial agent or a combination of both.

10. Sling according to any one of the preceding claims, **characterized in that** said sling has two end parts having cone-shaped subunits.

11. Sling according to any one of claims 1 to 9 **characterized in that** said sling has two and parts having multispheric shaped subunits.

12. Sling according to any one of claims 1 to 11 **characterized in that** the middle part of the sling has perforations throughout its surface, the shape of the perforations being selected in the group formed by the oval, rectangular, square, circular or any combination of these shapes.

13. Sling according to claim 12 **characterized in that** the perforation are symmetrically or randomly distributed.

14. Kit containing, in a sterile manner, at least one sling as defined in any one of Claims 1 to 13.

## Patentansprüche

1. Schlinge (1) zur Behandlung von Harninkontinenz, wobei die Schlinge aus einem Band besteht, das einen Mittelteil (2) und zwei Endteile (3) aufweist, wobei der Mittelteil durchlöchert ist und **dadurch gekennzeichnet, dass** die beiden Endteile ein Selbstfixiersystem bilden und dass die beiden Endteile aus mehreren Untereinheiten bestehen, wobei die Gestalt dieser Untereinheiten aus der Gruppe ausgewählt ist, zu der eine konische, dreieckige, rechteckige, quadratische, trapezförmige, rombische, ovale, zylindrische oder multisphärische Gestalt gehört.

2. Schlinge nach Anspruch 1, **dadurch gekennzeichnet, dass** die Basis der Untereinheiten gerade oder angelhakenförmig gestaltet ist.

3. Schlinge nach einem beliebigen der vorhegehenden Ansprüche, **dadurch gekennzeichnet, dass** der Mittelteil (2) aus einem biokompatiblen biologischen oder einem biokompatiblen synthetischen Material hergestellt ist.

4. Schlinge nach Anspruch 3, **dadurch gekennzeichnet, dass** der Mittelteil (2) aus synthetischen Materialien, wie beispielsweise Polyestern, Polypropylenen, Polyurethanen, Polyamiden, Nylons, Silikonen, Polytetrafluorethylenen, beispielsweise Teflon, Polyethylenterephthalaten, Latex oder irgendwelchen sonstigen thermogehärteten oder thermogeformten Kunststoffen oder Gummis, oder aus biologischen Materialien, wie beispielsweise Rinderpericardium, unterschiedlichen Arten von Kollagen, oder aus sonstigen tierischen oder menschlichen Derivaten, wie beispielsweise verarbeiteter Fascia Lata, kleiner Schweinedarm-Submukosa, Geweberegenerationsmatrix, hergestellt ist.

5. Schlinge nach Anspruch 4, **dadurch gekennzeichnet, dass** der Mittelteil (2) aus Silikon hergestellt ist.

6. Schlinge nach einem beliebigen der vorhergehenden Ansprüche, **dadurch gekennzeichnet, dass** der Mittelteil eine Verstärkung enthält, die vorzugsweise ein Polyestergewebe aufweist.

7. Schlinge nach einem beliebigen der vorhergehenden Ansprüche, **dadurch gekennzeichnet, dass** die Endteile aus einem Material, wie beispielsweise Polyester, Polypropylen, Polyurethan, Polyamid, Nylon, Silikon, Polytetrafluorethylen, beispielsweise Teflon, Polyethylenterephthalat, Latex oder aus irgendwelchen sonstigen thermogehärteten oder thermogeformten Kunststoffen oder Gummis hergestellt sind.

8. Schlinge nach einem beliebigen der vorhergehenden Ansprüche, **dadurch gekennzeichnet, dass** wenigstens einer der Endteile eine Lochung enthält.

9. Schlinge nach einem beliebigen der vorhergehenden Ansprüche, **dadurch gekennzeichnet, dass** sie vollständig oder teilweise mit einem antibiotischen, einem antimikrobiellen Mittel oder einer Kombination von beiden imprägniert oder überzogen ist.

10. Schlinge nach einem beliebigen der vorhergehenden Ansprüche, **dadurch gekennzeichnet, dass** die Schlinge zwei Endteile mit konischen Untereinheiten aufweist.

11. Schlinge nach einem beliebigen der Ansprüche 1 bis 9, **dadurch gekennzeichnet, dass** die Schlinge zwei Endteile mit multisphärisch gestalteten Untereinheiten aufweist.

12. Schlinge nach einem beliebigen der Ansprüche 1 bis 11, **dadurch gekennzeichnet, dass** der Mittelteil der Schlinge in seiner gesamten Oberfläche Löcher aufweist, wobei die Gestalt der Löcher aus der Gruppe ausgewählt ist, die durch ovale, rechteckige, quadratische, kreisförmige Formen oder eine beliebige Kombination dieser Formen gebildet ist.

13. Schlinge nach Anspruch 12, **dadurch gekennzeichnet, dass** die Löcher symmetrisch oder zufällig verteilt sind.

14. Ausstattung, die in einer sterilen Weise wenigstens eine Schlinge enthält, wie sie in einem beliebigen der Ansprüche 1 bis 13 definiert ist.

## Revendications

1. Bandelette (1) conçue pour le traitement de l'incontinence urinaire, constituée d'une bande qui comprend une partie médiane (2) et deux parties terminales (3), laquelle partie médiane est perforée, et **caractérisée en ce que** les deux parties terminales constituent un système autofixant et **en ce que** ces deux parties terminales sont constituées de multiples sous-unités dont la forme est choisie parmi les formes conique, triangulaire, rectangulaire, carrée, trapézoïdale, rhomboïdale, ovale, cylindrique et multisphérique.

2. Bandelette conforme à la revendication 1, **caractérisée en ce que** la base des sous-unités est rectiligne ou en forme de hameçon.

3. Bandelette conforme à l'une des revendications précédentes, **caractérisée en ce que** la partie médiane (2) est faite en un matériau biologique biocompatible ou en un matériau synthétique biocompatible.

4. Bandelette conforme à la revendication 3, **caractérisée en ce que** la partie médiane (2) est faite en un matériau synthéti-que, tels les polyesters, polypropylènes, polyuréthanes, polyamides, nylons, silicones, polytétrafluoroéthylènes comme le Téflon, poly(éthylène téréphta-late), latex et autres matières plastiques ou gommes thermodurcies ou thermoformées, ou en un matériau biologique comme du péricarde de bovin, des collagènes de divers types, ou d'autres matériaux d'origine humaine ou animale comme un fascia lata traité, une sous-muqueuse d'intestin grêle de porc ou une matrice de régénération de tissus.

5. Bandelette conforme à la revendication 4, **caractérisée en ce que** la partie médiane (2) est en silicone.

6. Bandelette conforme à l'une des revendications précédentes, **caractérisée en ce que** la partie médiane comporte un renfort, constitué de préférence d'un morceau de.toile en polyester.

7. Bandelette conforme à l'une des revendications précédentes, **caractérisée en ce que** les parties terminales sont faites en un matériau comme les polyesters, polypropylènes, polyuréthanes, polyamides, nylons, silicones, polytétrafluoroéthylènes comme le Téflon, poly(éthylène téréphtalate), latex et autres matières plastiques ou gommes thermodurcies ou thermoformées.

8. Bandelette conforme à l'une des revendications précédentes, **caractérisée en ce qu'**au moins l'une des parties terminales présente une perforation.

9. Bandelette conforme à l'une des revendications précédentes, **caractérisée en ce qu'**elle est entièrement ou partiellement imprégnée ou enduite d'un antibiotique, d'un agent anti-microbien ou d'une combinaison d'agents de ces deux types.

10. Bandelette conforme à l'une des revendications précédentes, **caractérisée en ce qu'**elle présente deux parties terminales comportant des sous-unités de forme conique.

11. Bandelette conforme à l'une des revendications 1 à 9, **caractérisée en ce qu'**elle présente deux parties terminales comportant des sous-unités de forme multisphérique.

12. Bandelette conforme à l'une des revendications 1 à 11, **caractérisée en ce que** la partie médiane de la bandelette présente, sur toute sa surface, des perforations dont la forme est choisie dans l'ensemble constitué par les formes ovale, rectangulaire, carrée, circulaire, ainsi que toute combinaison de telles formes.

13. Bandelette conforme à la revendication 12, **caractérisée en ce que** les perforations sont réparties de manière symétrique ou aléatoire.

14. Trousse contenant, dans des conditions stériles, au moins une bandelette conforme à l'une des revendications 1 à 13.
